# EUROPÄISCHE PATENTANMELDUNG

(11) **EP 2 594 241 A1**
(43) Veröffentlichungstag der Anmeldung: **22.05.2013**
(21) Anmeldenummer: 12184608.3
(22) Anmeldetag: 17.09.2012
(51) Int. Cl.: A61G 12/00, A61B 19/02

(54) **Stationswagen, insbesondere Visiten- und Pflegewagen**

(30) Priorität: 15.11.2011 DE 202011051973 U
(71) Anmelder: Optiplan Gesellschaft für optische Planungsgeräte mit beschränkter Haftung, 40489 Düsseldorf (DE)
(72) Erfinder: Lerner, Sabine, 40489 Düsseldorf (DE); Wagner, Kai, 40489 Düsseldorf (DE)
(74) Vertreter: Stenger, Watzke & Ring

(57) **Zusammenfassung**

Die Erfindung betrifft einen Stationswagen, insbesondere Visiten- und Pflegewagen für Krankenhäuser, Pflegeheime und sonstige Einrichtungen des Gesundheitswesens, mit einem fahrbar ausgebildeten Schrankunterteil (2), das der Aufnahme von Utensilien dient, wobei ein Schrankoberteil (3), in Höhenrichtung (7) relativ verfahrbar zum Schrankunterteil (2) ausgebildet ist.

## Beschreibung

Die Erfindung betrifft einen Stationswagen, insbesondere einen Visiten- und Pflegewagen für Krankenhäuser, Pflegeheime und sonstige Einrichtungen des Gesundheitswesens, mit einem fahrbar ausgebildeten Schrankunterteil, das der Aufnahme von Utensilien dient.

Ein Stationswagen der eingangs genannten, d. h. gattungsgemäßen Art ist aus dem Stand der Technik an sich gut bekannt. Derlei Wagen sind beispielsweise aus der DE 297 14 981, der DE 20 2005 013 115, der DE 20 2005 013 114 sowie der DE 20 2008 015 373 bekannt geworden.

Obgleich sich Wagen, insbesondere Visiten- und Pflegewagen der vorgenannten Art im alltäglichen Praxiseinsatz bewährt haben, besteht ein ständiges Bestreben danach, eine mit Bezug auf den bestimmungsgemäßen Verwendungsfall optimierte Ausgestaltung bereitzustellen. Es ist deshalb die Aufgabe der Erfindung, einen gegenüber dem Stand der Technik neuartigen Wagen, insbesondere Pflegewagen bereitzustellen, der in seiner Handhabung Verbesserungen bietet.

Zur Lösung dieser Aufgabe wird mit der Erfindung ein Wagen der eingangs genannten Art vorgeschlagen, der sich auszeichnet durch ein Schrankoberteil, das in Höhenrichtung relativ verfahrbar zum Schrankunterteil ausgebildet ist.

Der Wagen, insbesondere Visiten- und Pflegewagen nach der Erfindung verfügt über ein Schrankunterteil einerseits und ein Schrankoberteil andererseits. Dabei ist das Oberteil in Höhenrichtung des Wagens oberhalb des Schrankunterteils angeordnet. Die erfindungsgemäße Besonderheit besteht nun darin, dass das Schrankoberteil in seiner relativen Lage gegenüber dem Schrankunterteil verfahrbar ist, d. h. ein Verfahren des Schrankoberteils in Höhenrichtung relativ gegenüber dem Schrankunterteil stattfinden kann.

Die mit der Erfindung vorgeschlagene Konstruktion gestattet in vorteilhafterweise eine individuelle Einstellung der Höhenlage des Schrankoberteils. Dies erweist sich insbesondere aus ergonomischen Gründen von Vorteil, da je nach bestimmungsgemäßem Verwendungsfall eine individuelle Höheneinstellung des Schrankoberteils vorgenommen werden kann. Aus dem Stand der Technik vorbekannte Visiten- und Pflegewagen der eingangs genannten Art bieten diese Einstellmöglichkeit nicht.

Die Höhenverstellbarkeit des Schrankoberteils kann insbesondere dazu genutzt werden, eine funktionsgerechte Anpassung an eine in einem Bett liegende Bedienperson einerseits und an eine in einem Sitzmöbel sitzende Bedienperson andererseits vornehmen zu können. Es ist eine Verwendung des erfindungsgemäßen Wagens auch als Stehpult einerseits sowie als einem Tisch beigeordneter Rollcontainer andererseits möglich.

Der Höhenabstand zwischen dem Schrankoberteil und dem Schrankunterteil kann durch ein Verfahren des Schrankoberteils relativ gegenüber dem Schrankunterteil individuell eingestellt werden. Dem Bedienpersonal ist es so gestattet, eine auf den individuellen Anwendungsfall bezogene Höheneinstellung des Schrankoberteils in Relation zum Schrankunterteil vornehmen zu können.

Das Schrankoberteil wird gemäß einem besonderen Vorschlag der Erfindung von einer Säule getragen. Dabei kann entweder vorgesehen sein, dass das Schrankoberteil in Längsrichtung der Säule verfahrbar von der Säule getragen ist oder dass es sich bei der Säule um eine teleskopierbare Säule handelt. Gemäß erst genannter Alternative erfolgt eine Höhenverstellung dadurch, dass das Schrankoberteil in Längsrichtung der Säule verfahren und in einer gewünschten Stellung in seiner relativen Lage gegenüber der Säule positioniert wird. Im zweitgenannten Fall erfolgt eine Höhenverstellung des Schrankoberteils durch eine Teleskopierbewegung der Säule.

Die Säule ist mit ihrem dem Schrankoberteil gegenüberliegenden Endabschnitt am Schrankunterteil angeordnet. Insofern bilden das Schrankunterteil, die Säule und das Schrankoberteil eine gemeinsame Einheit. Diese ist verfahrbar ausgebildet, was eine freie Positionierung im Raum gestattet.

Gemäß einer bevorzugten Ausführungsform der Erfindung dient der Wagen der Aufnahme von elektrischen Geräten, insbesondere zur Aufnahme eines Rechners. In diesem Fall ist das Schrankunterteil für die Aufnahme des eigentlichen Rechners vorgesehen. Das Schrankoberteil dient indes insbesondere der Aufnahme von Rechnerbedieneinheiten, wie z. B. einer Tastatur, einer Maus und/oder dergleichen Peripheriegeräte. Zum Zwecke der elektrischen und/oder elektronischen Verbindung der einzelnen Bestandteile bzw. Komponenten einer Rechneranlage ist ein Kabelkanal vorgesehen, der bevorzugterweise im Inneren der Säule verlaufend ausgebildet ist. In vorteilhafter Weise kann so eine völlig versteckte Anordnung von Kabeln erfolgen, was einerseits aus hygienischen Gründen und andererseits aus sicherheitsrelevanten Gründen von Vorteil ist.

Gemäß einem weiteren Merkmal der Erfindung ist vorgesehen, dass das Schrankoberteil mit einem Monitorhalter ausgerüstet ist. Im bestimmungsgemäßen Verwendungsfall dient dieser Halter der Aufnahme bzw. Anordnung eines Monitors, eines Flachbildschirms und/oder dergleichen optischen Anzeigeeinrichtung. Dabei ist bevorzugterweise vorgesehen, dass der Monitorhalter eine relativ zum Schrankoberteil verschwenkbare Anordnung eines Monitors gestattet. Es ist mithin eine Ausrichtung des Monitors relativ gegenüber dem Schrankoberteil und damit relativ gegenüber dem gesamten Wagen möglich. In Kombination mit der Höhenverstellmöglichkeit des den Monitorhalter tragenden Schrankoberteils kann verwenderseitig eine individuellen Wünschen gerecht werdende Ausrichtung des Monitorhalters bzw. des davon getragenen Monitors vorgenommen werden.

Es ist gemäß einem weiteren Merkmal der Erfindung vorgesehen, dass das Schrankoberteil einen nach Art einer Schublade aus dem Schrankoberteil heraus verfahrbaren Auszug bereitstellt. Dieser Auszug kann insbesondere zur Aufnahme einer Tastatur dienen. Im Verwendungsfall wird sie mit Hilfe des Auszugs aus dem Schrankoberteil heraus verfahren und ist für einen Verwender frei zugänglich. Im Nicht-Verwendungsfall kann sie platzsparend innerhalb des Schrankoberteils durch einfaches Einfahren des Auszuges angeordnet werden.

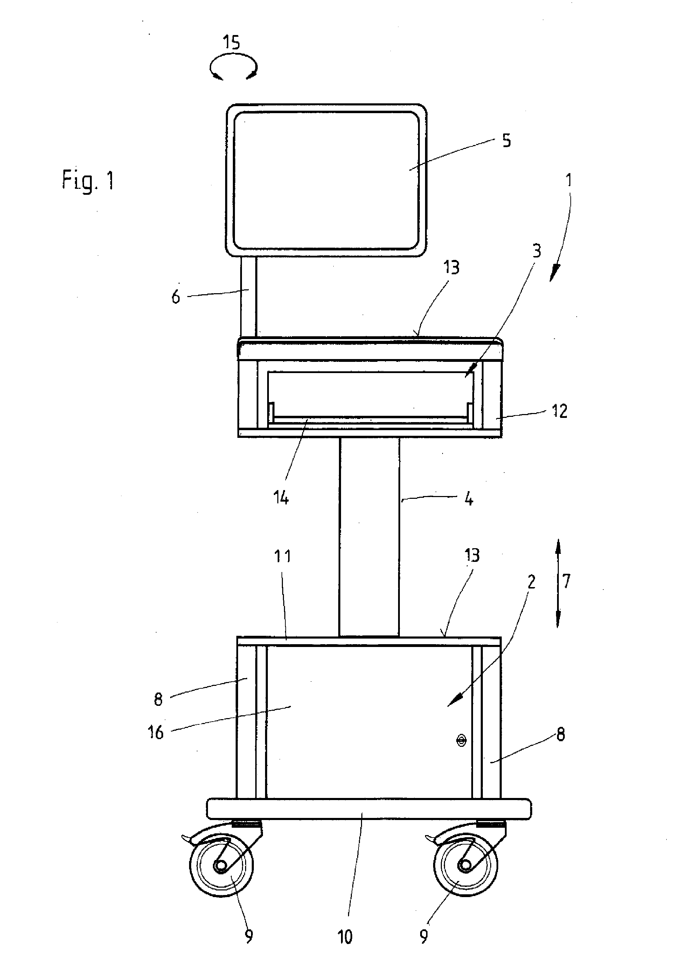

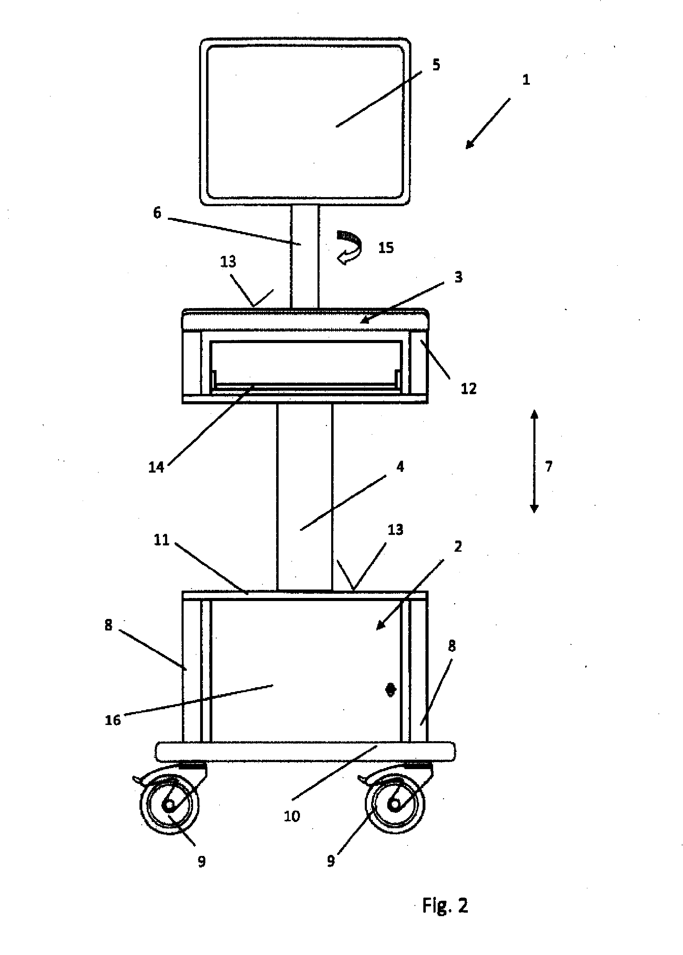

Weitere Merkmale und Vorteile der Erfindung ergeben sich aus der nachfolgenden Beschreibung anhand der Figuren 1 und 2, die in schematischer Frontansicht jeweils einen Wagen nach der Erfindung in der Ausgestaltung eines Pflegewagens für ein Krankenhaus zeigen.

Der erfindungsgemäße Stationswagen 1 verfügt über ein Schrankunterteil 2. Dieses Schrankunterteil 2 verfügt über Pfosten 8, die der Anordnung und gegenseitigen Abstützung von Seitenwänden dienen. Frontseitig verfügt das Schrankunterteil 2 über eine verschwenkbar ausgebildete Flügeltür 16.

Die Pfosten 8 tragen fußbodenseitig Tragrollen 9, womit der Stationswagen 1 insgesamt verfahrbar ausgebildet ist. Das Schrankunterteil 2 kann zudem mit einem umlaufenden Rammschutz 10 ausgerüstet sein, wie das Ausführungsbeispiel nach Figur 1 erkennen lässt.

Oberseitig schließt das Schrankunterteil 2 mit einer Deckplatte 11 ab, die ihrerseits eine Ablagefläche 13 bereitstellt.

Der Stationswagen 1 verfügt des Weiteren über eine Säule 4. Diese ist in Höhenrichtung 7 verlaufend ausgerichtet und am Schrankunterteil 2 angeordnet.

Die Säule 4 trägt ein Schrankoberteil 3. Erfindungsgemäß wird das Schrankoberteil 3 in Längsrichtung der Säule 4, d. h. in Höhenrichtung 7 verfahrbar von der Säule 4 getragen. In der Konsequenz ist das Schrankoberteil 3 in Höhenrichtung 7 relativ verfahrbar zum Schrankunterteil 2 ausgebildet. Es kann also eine individuellen Wünschen gerecht werdende Ausrichtung des Schrankoberteils 3 in Höhenrichtung 7 relativ gegenüber dem Schrankunterteil 2 stattfinden.

Das Schrankoberteil 3 ist nach Art eines einseitig offenen Kastenteils 12 ausgebildet. Es stellt oberseitig eine Ablagefläche 13 bereit und verfügt über einen nach Art einer Schublade aus dem Schrankoberteil 3 heraus verfahrbaren Auszug 14. Dieser Auszug 14 kann insbesondere der Aufnahme einer Tastatur einer Rechneranlage dienen.

Das Schrankoberteil 3 ist des Weiteren mit einem Monitorhalter 6 ausgerüstet. Dieser Monitorhalter 6 trägt einen Monitor 5, wobei der Monitorhalter 6 den Monitor 5 in Pfeilrichtung 15 verschwenkbar aufnimmt. Gemäß der Ausführungsform nach Fig. 1 ist der Monitorhalter 6 seitlich des Schrankoberteils 3 ausgebildet. Die Ausführungsform nach Fig. 2 zeigt eine mittige Ausgestaltung des Monitorhalters 6.

Die Säule 4 nimmt bevorzugterweise einen im Säuleninneren verlaufenden Kabelkanal auf. Kabel, insbesondere elektrische Verbindungskabel können so versteckt geführt werden.

Gemäß eines besonderen Verwendungsfalls kann der erfindungsgemäße Visiten- und Pflegewagen 1 als Rechnerwagen dienen. In diesem Fall dient das Schrankunterteil 2 der Aufnahme einer Rechnereinheit. Das Schrankoberteil 3 dient der Aufnahme von Bedieneinheiten, wie z. B. einer Tastatur oder einer Maus. Eine Kabelverbindung zwischen diesen Komponenten der Rechneranlage erfolgt über den Kabelkanal innerhalb der Säule 4. Die Verbindungskabel zum Monitor 5 werden gleichfalls versteckt geführt, und zwar über den Monitorhalter 6, das Schrankoberteil 3 und die Säule 4 bis hin zum Schrankunterteil 2.

### Bezugszeichenliste

- 1: Stationswagen
- 2: Schrankunterteil
- 3: Schrankoberteil
- 4: Säule
- 5: Monitor
- 6: Monitorhalter
- 7: Höhenrichtung
- 8: Pfosten
- 9: Tragrolle
- 10: Rammschutz
- 11: Deckplatte
- 12: Kastenteil
- 13: Ablagefläche
- 14: Auszug
- 15: Pfeil
- 16: Tür

## Patentansprüche

1. Stationswagen, insbesondere Visiten- und Pflegewagen für Krankenhäuser, Pflegeheime und sonstige Einrichtungen des Gesundheitswesens, mit einem fahrbar ausgebildeten Schrankunterteil (2), das der Aufnahme von Utensilien dient, **gekennzeichnet durch** ein Schrankoberteil (3), das in Höhenrichtung (7) relativ verfahrbar zum Schrankunterteil (2) ausgebildet ist.

2. Stationswagen nach Anspruch 1, **gekennzeichnet durch** eine das Schrankoberteil (3) tragende Säule (4).

3. Stationswagen nach Anspruch 2, **dadurch gekennzeichnet, dass** das Schrankoberteil (3) in Längsrichtung der Säule (4) verfahrbar von der Säule (4) getragen ist.

4. Stationswagen nach Anspruch 2, **dadurch gekennzeichnet, dass** die Säule (4) teleskopierbar ausgebildet ist.

5. Stationswagen nach einem der vorhergehenden Ansprüche 2 bis 4, **dadurch gekennzeichnet, dass** die Säule (4) mit ihrem dem Schrankoberteil (3) gegenüberliegenden Endabschnitt am Schrankunterteil (2) angeordnet ist.

6. Stationswagen nach einem der vorhergehenden Ansprüche 1 bis 5, **dadurch gekennzeichnet, dass** die Säule (4) einen im Inneren der Säule (4) verlaufenden Kabelkanal aufweist.

7. Stationswagen nach der vorhergehenden Ansprüche, **dadurch gekennzeichnet, dass** das Schrankoberteil (3) mit einem Monitorhalter (6) ausgerüstet ist.

8. Stationswagen nach Anspruch 7, **dadurch gekennzeichnet, dass** der Monitorhalter (6) eine relativ zum Schrankoberteil (3) verschwenkbare Anordnung eines Monitors (5) gestattet.

9. Stationswagen nach einem der vorhergehenden Ansprüche, **dadurch gekennzeichnet, dass** das Schrankoberteil (3) einen nach Art einer Schublade aus dem Schrankoberteil (3) heraus verfahrbaren Auszug (14) bereitstellt.
